# FASCICULE DE BREVET EUROPEEN

(11) **EP 2 191 003 B1**
(45) Date de publication et mention de la délivrance du brevet: **03.06.2015**
(21) Numéro de dépôt: 08837762.7
(22) Date de dépôt: 17.09.2008
(51) Int. Cl.: C12P 7/44, C07C 51/36, C07C 55/02, C07C 69/593, C07C 57/13, C07C 69/34, C07C 67/333, C07C 67/343, C12P 7/62, C07C 67/303, C07C 51/353

(54) **PROCÉDÉS DE SYNTHÈSE DE DIACIDES GRAS PAR MÉTATHÈSE DE DIACIDES INSATURÉS OBTENUS PAR FERMENTATION D'ACIDES GRAS NATURELS**
VERFAHREN ZUR SYNTHESE VON DIFETTSÄUREN DURCH METATHESE VON DURCH FERMENTIERUNG NATÜRLICHER FETTSÄUREN GEWONNENEN UNGESÄTTIGTEN DISÄUREN
METHODS FOR THE SYNTHESIS OF FATTY DIACIDS BY THE METATHESIS OF UNSATURATED DIACIDS OBTAINED BY FERMENTATION OF NATURAL FATTY ACIDS

(30) Priorité: 20.09.2007 FR 0757691
(43) Date de publication de la demande: 02.06.2010
(73) Titulaire: ARKEMA FRANCE, 92700 Colombes (FR)
(72) Inventeur: DUBOIS, Jean-Luc, F-69390 Millery (FR)
(86) Numéro de dépôt international: PCT/FR2008/051664
(87) Numéro de publication internationale: WO 2009/047444

(56) Documents cités:
- US-A- 4 474 882
- C.-X. BAI ET AL.: "Lewis-acid assisted cross metathesis of acrylonitrile with functionalized olefins catalyzed by phosphine-free ruthenium carbene complex." ORG.BIOMOL.CHEM., vol. 3, 2005, pages 4139-4142, XP002481691 cité dans la demande
- A.K. CHATTERJEE ET AL.: "Formal vinyl CH activation and allylic oxidation by olefin metathesis" ANGEW. CHEM. INT.ED., vol. 41, no. 17, 2002, pages 3171-3174, XP002481692
- DATABASE BEILSTEIN BEILSTEIN INSTITUTE FOR ORGANIC CHEMISTRY, FRANKFURT-MAIN, DE; XP002481693 Database accession no. 1780726
- DATABASE BEILSTEIN BEILSTEIN INSTITUTE FOR ORGANIC CHEMISTRY, FRANKFURT-MAIN, DE; XP002481694 Database accession no. 1707959

## Description

L'invention vise un procédé de synthèse par métathèse croisée de diacides ou de diesters gras à chaînes courtes saturés ou non à partir d'un acide gras ou d'un ester gras mono-insaturé naturel.

Les diacides ou diesters insaturés les plus connus sont ceux comportant des chaînes comportant de 4 à 6 atomes de carbone tels que les acides en C4, acides maléique et fumarique, les acides en C5, acides citraconique, mesaconique et itaconique et les acides en C6, les acides 2-méthylène glutarique et muconique. En revanche en ce qui concerne les diacides à chaîne longue, les seuls ayant une certaine importance sont des dimères généralement obtenus par condensation d'acides carboxyliques insaturés. Les propriétés, synthèses et utilisations de ces diacides sont décrites dans l'Encyclopédie Uhlmann Vol. A8, pages 533-536.

Les diacides saturés sont obtenus industriellement selon différentes méthodes, mais qui toutes présentent certains inconvénients. Un large panorama de ces méthodes est développé sous la référence précédente aux pages 523-536.

On peut y distinguer les méthodes par dégradation telles que l'ozonolyse ou l'oxydation des acides gras végétaux.

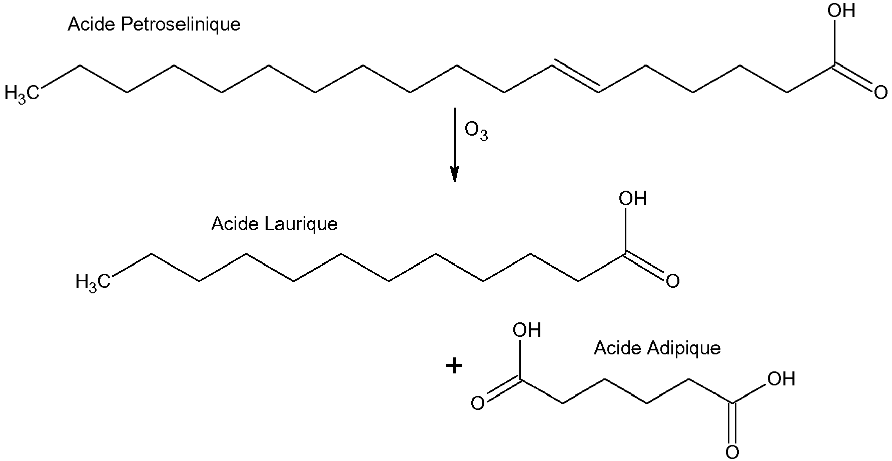

L'ozonolyse de l'acide oléique, de l'acide pétrosélinique et de l'acide érucique permet de produire respectivement les diacides à 9, 6 et 13 atomes de carbone selon le processus réactionnel ci-dessus pour l'acide pétrosélinique.

Un autre exemple est le clivage de l'acide ricinoléique par action de la soude à une température supérieure à 180°C. Cette méthode utilisée industriellement permet d'obtenir le diacide à 10 atomes de carbone. La même méthode, telle qu'illustrée dans le schéma ci-dessous peut être appliquée à l'acide lesquérolique et conduit à la formation d'un diacide à 12 atomes de carbone. Cette méthode présente l'avantage d'utiliser des matières premières renouvelables mais est limitée essentiellement au diacide en C10, l'acide lesquérolique étant encore peu répandu, et donc cette méthode est relativement peu utilisée.

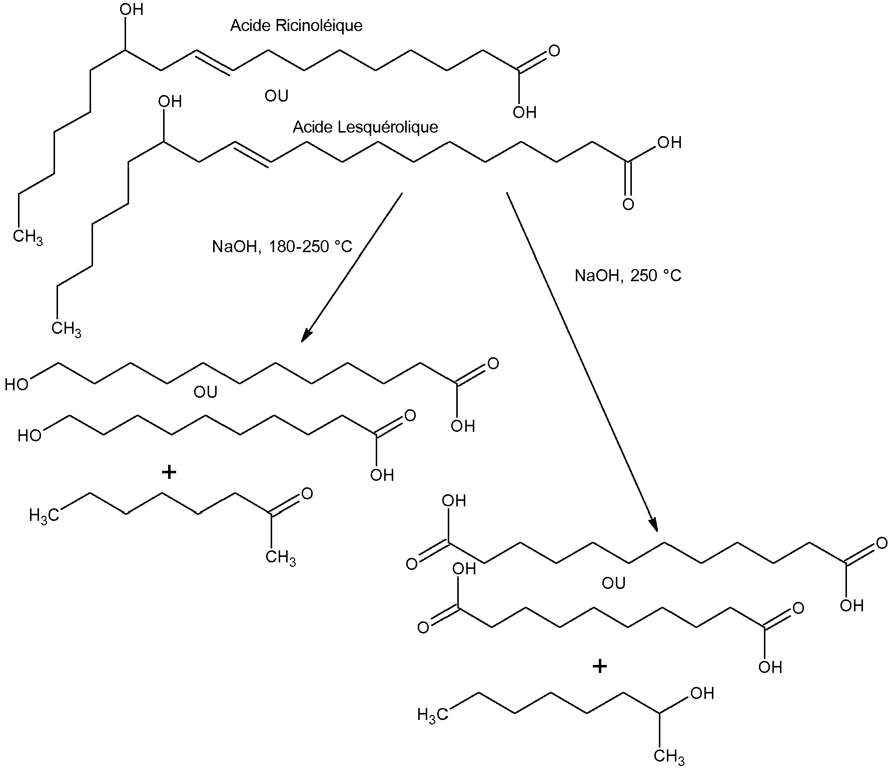

On peut citer aussi la dégradation oxydante des acides monocarboxyliques par action de N₂O₄. L'oxydation de l'acide stéarique permet d'obtenir un mélange d'acide sébacique et d'acide caprylique ; à partir d'acide palmitique on peut obtenir l'acide subérique.

Il est également possible d'obtenir des diacides à partir de molécules de plus petites tailles en utilisant des techniques variantes de la carbonylation.

Enfin on peut citer la fermentation par une levure, un champignon ou une bactérie de substrats hydrocarbonés paraffines, acides ou esters gras saturés ou non qui permet d'obtenir une oxydation des composés du substrat. Cette méthode est bien connue. Elle est notamment illustrée dans l'article de W. H. Eschenfeldt et al. « Transformation of fatty Acids Catalyzed by Cytochrome P450 Monooxygenase Enzymes of *Candida tropicalis* » et les brevets FR 2,445,374 US4,474,882, US 3,823,070, US 3,912,586, US 6,660,505, US 6,569,670 et 5,254,466. Elle permet d'obtenir de nombreux diacides de longueur de chaîne variable.

Dans l'industrie chimique et notamment celle des polymères, telle que la production de polyamides de type diacides-diamines ou de polymères techniques, il est nécessaire de disposer de toute une gamme de diacides saturés ou insaturés. Ces diacides constitueront des matières premières qui pourront en outre être convertis en diamines de même longueur de chaîne par simple réaction chimique. Les diacides insaturés seront utilisés comme monomères de polymères de spécialités.

Il est donc nécessaire de trouver un type de procédé qui permet d'obtenir une gamme quasiment complète de diacides saturés ou non qui, en outre, utilise des matières renouvelables d'origine naturelle.

L'objectif de l'invention est un procédé de production de toute une gamme de diacides ou diesters, saturés ou non, de formule générale : ROOC-(CH2)n-(CH=CH)a-(CH2)m-COOR1 à partir d'acides gras d'origine naturelle.

Dans la suite de la présente description on utilisera pour la clarté de l'exposé le terme diacide pour désigner indifféremment le diacide et le diester. En effet dans le procédé de l'invention, on peut traiter l'acide gras soit sous sa forme acide soit sous sa forme ester. Le passage d'une forme à l'autre s'effectue simplement par alcoolyse, estérification ou hydrolyse.

La solution proposée consiste à travailler à partir des acides gras naturels à longue chaîne mono-insaturés que l'on oxyde par fermentation en diacides que l'on soumet à une métathèse croisée avec un composé de type acrylique.

On entend par acide gras naturel à longue chaîne un acide issu des milieux végétal ou animal, y compris les algues, plus généralement du règne végétal, et donc renouvelable comportant au moins 10 et de préférence au moins 14 atomes de carbone par molécule.

On peut citer à titre d'exemples de tels acides, les acides en C10, les acides obtusilique (cis-4-décénoique) et caproléique (cis-9-décénoique), les acides en C12, les acides lauroléique (cis-5-dodécénoique) et lindérique (cis-4-dodécénoique), les acides en C14, les acides myristoléique (cis-9-tétradécénoique), physétérique (cis-5-tetradécénoique) et tsuzuique (cis-4-tetradécénoique), l'acide en C16, l'acide palmitoléique (cis-9- hexadécénoique), les acides en C18, les acides oléique (cis-9-octadécénoique), élaïdique (trans-9-octadécénoique), pétrosélinique (cis-6-octadécénoique), vaccénique (cris-11-octadécénoique) et ricinoléique (12-hydroxy-cis-9-octadécénoique), les acides en C20, les acides gadoléique (cis-9-eicosénoique), gondoique (cis-11-eicosénoique), l'acide cis-5-eicosènoique et l'acide lesquérolique (14-hydroxy-cis-11-eicosénoique), les acides en C22, les acides cétoléique (cis-11-docosénoique) et érucique (cis-13-docosénoique).

Ces divers acides sont issus des huiles végétales extraites de diverses plantes telles que le tournesol, le colza, le ricin, le lesquerella, l'olive, le soja, le palmier, la coriandre, le céleri, l'aneth, la carotte, le fenouil, le Limnanthes Alba (meadowfoam).

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise la synthèse d'acides ou d'esters gras à courtes chaînes. Dans la présente demande les diacides ou les diesters gras à courtes chaînes désignent des molécules comportant dans la chaîne principale de 6 à 16 atomes de carbones adjacents mais qui sont synthétisés à partir d'un diacide supérieur et ayant une longueur de chaîne principale dont le rapport avec celle de l'acide ou ester gras insaturé de départ est compris entre 0,35 et 0,9, de préférence entre 0,4 et 0,8, et de manière encore plus préférée entre 0,5 et 0,7.

L'invention vise un procédé de synthèse de diacides ou de diesters de formule générale : ROOC-(CH₂)ₙ(CH=CH)ₐ(CH₂)ₘCOOR₁ dans laquelle n et m différents représentent chacun un nombre entier tel que leur somme est comprise entre 6 et 15, a un indice égal à 0 ou 1 et R et R₁ sont soit H, soit un radical alkyle de 1 à 4 atomes de carbone, à partir d'acides ou d'esters gras naturels à longue chaîne mono-insaturés comportant au moins 10 atomes de carbone adjacents par molécule, de formule CH₃-(CH₂)ₚ-CH=CH-(CH₂)_{q}-COOR, dans laquelle R représente H ou un radical alkyle comportant de 1 à 4 atomes de carbone, et p et q identiques ou différents, sont des indices compris entre 2 et 11, consistant dans une première étape, à oxyder par fermentation en présence d'un micro-organisme ledit acide ou ester gras naturel en au moins un diacide ou diester carboxylique mono-insaturé puis à soumettre dans une deuxième étape le produit de la première étape à une métathèse croisée avec un composé de formule R₂OOC-(CH₂)ₓ-CH=CH-R₃ dans laquelle R₂ est soit H, soit un radical alkyle comportant de 1 à 4 atomes de carbone, x est 0, 1 ou 2 et R₃ est H, CH₃ ou COOR₂, formant dans ce dernier cas une molécule cyclique ou non, pour obtenir un composé insaturé de formule ROOC-(CH₂)_{q}-CH=CH-(CH₂)ₓ COOR₂, puis dans une troisième étape, facultative, enfin à transformer par hydrogénation de la double liaison le composé insaturé en composé saturé.

La métathèse croisée est faite avec l'acide acrylique quand R₂=H, x=0 et R₃=H. Dans le cas où x=1, R₂=H et R₃=CH₃, le composé est HOOC-CH₂-CH=CH-CH₃ et est obtenu par exemple par hydroxycarbonylation du butadiène. Dans ce cas, au cours de la métathèse croisée, du propylène est produit et est éliminé du milieu réactionnel.

De préférence, lorsque R₃ est COOR₂, R₂OOC-(CH₂)ₓ-CH=CH-R₃ est une molécule symétrique avec x=0. Lorsque R₃ est CH₃, R₂OOC-(CH₂)ₓ-CH=CH-R₃ réagit avec un acide gras par métathèse croisée, la réaction conduit à un diacide et un acide gras plus court, mais aussi à du propylène. Le propylène est éliminé au fur et à mesure du milieu réactionnel ce qui déplace la réaction vers les produits souhaités.

Lorsque R₂OOC-(CH₂)ₓ-CH=CH-COOR₂ forme une molécule cyclique comme l'anhydride maléique, alors la métathèse croisée conduit à un acide gras insaturé contenant aussi une fonction anhydride. Le diacide et l'acide gras peuvent être libérés par hydrolyse.

Dans le procédé de l'invention on utilise des acides ou esters gras d'origine naturelle, c'est-à-dire ceux contenus dans des huiles ou des graisses. Ces dernières sont en fait constituées à côté de l'ester ou de l'acide entrant dans la réaction d'un mélange d'esters ou acides de formules voisines. A titre d'exemples l'huile de tournesol contient outre l'acide oléique de l'acide linoléique; l'huile de ricin contient outre l'acide ricinoléique à la fois de l'acide oléique et l'acide linoléique, l'huile de colza contient outre l'acide oléique à la fois de l'acide linoléique, de l'acide linolénique et de l'acide gadoléique. La présence de ces acides di-insaturés ou poly-insaturés n'a pas de conséquence importante sur le déroulement du procédé dans la mesure où lors de l'étape suivant la réaction de métathèse une séparation des produits est effectuée.

La première étape est réalisée par fermentation en présence d'un micro-organisme c'est-à-dire au moyen de toute bactérie, champignon ou levure permettant l'oxydation de l'acide ou ester gras de la charge. Cette fermentation pourra être réalisée notamment en présence de micro-organismes comportant des enzymes oxydantes de type Oxygenase. Elle pourra être réalisée, par exemple, en présence d'une souche de *Candida tropicalis* contenant les enzymes Cytochrome P450 Monooxygenase telles que celles décrites dans la publication de W. H. Eschenfeldt et al «Transformation of fatty Acids Catalyzed by Cytochrome P450 Monooxygenase Enzymes of Candida tropicalis» paru dans Applied and Environmental Microbiology, Oct. 2003 p. 5992-5999 et les brevets FR 2,445,374 US 4,474,882, US 3,823,070, US 3,912,586, US 6,660,505, US 6,569,670 et 5,254,466.

On peut indifféremment fermenter un acide gras, un ester gras et un triglycéride. En effet, les micro-organismes sont aussi capables de métaboliser les alcools et le glycérol.

Les réactions de métathèse mises en oeuvre en deuxième étape sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004 (Plenum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al J. Am. Chem. Soc. 108 (1986) 2771, ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que rhénium, molybdène et tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Dans le procédé de l'invention, le catalyseur de métathèse actif et sélectif est à base de ruthénium, éventuellement en combinaison avec le rhénium.

La deuxième étape est illustrée par des exemples de synthèse de diacides gras à courtes chaînes sont donnés ci après. Tous les mécanismes détaillés ci-dessous illustrent, pour faciliter l'exposé, la forme acide. Cependant, la métathèse est aussi efficace avec un ester et même souvent plus efficace. De la même manière, les schémas illustrent des réactions avec l'isomère cis des acides (ou esters) ; les mécanismes sont aussi bien applicables aux isomères trans.

Le processus réactionnel de cette deuxième étape mettant en oeuvre le diacide oléique et l'acide acrylique est le suivant :

HOOC-(CH₂)₇-CH=CH-(CH₂)₇-COOH + HOOC-CH=CH₂ ⇔ HOOC-CH=CH-(CH₂)₇-COOH + CH₂=CH-(CH₂)₇-COOH

et par réaction consécutive avec l'acide acrylique utilisé en excès :

CH₂=CH- (CH₂)₇-COOH + HOOC-CH=CH₂ ⇔ HOOC-CH=CH-(CH₂)₇-COOH + CH₂=CH₂

L'acide α-ω 2-undécènedioique peut si nécessaire être transformé en acide saturé α-ω undécanedioique par hydrogénation selon le processus suivant :

HOOC-CH=CH-(CH₂)₇-COOH + H₂ HOOC-(CH₂)₉-COOH

On peut observer qu'au cours de la réaction de métathèse croisée avec un excès d'acide acrylique se forme l'acide 9-décénoique qui va entraîner la formation par une nouvelle métathèse croisée avec l'acide acrylique le composé de formule HOOC-CH=CH-(CH₂)₇-COOH avec production d'éthylène. Un avantage important du procédé est ainsi l'absence de co-produit, hormis l'éthylène qui est facilement éliminé. Le mécanisme réactionnel de cette réaction est illustrée par le schéma 1 ci-dessous.

Dans certaines conditions de fermentation l'acide oléique est oxydé en acide 9-octadécénedioique.

Le processus réactionnel de cette deuxième étape mettant en oeuvre ce diacide et l'acide acrylique pour produire deux diacides identiques peut être décrit par le mécanisme suivant :

HOOC-(CH₂)₇-CH=CH-(CH₂)₇-COOH + HOOC-CH=CH₂ ⇔ HOOC-CH=CH-(CH₂)₇-COOH + CH₂=CH-(CH₂)₇-COOH

et par réaction consécutive avec l'acide acrylique utilisé en excès :

CH₂=CH-(CH₂)₇-COOH + HOOC-CH=CH₂ ⇔ HOOC-CH=CH-(CH₂)₇-COOH + CH₂=CH₂.

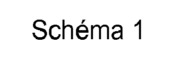

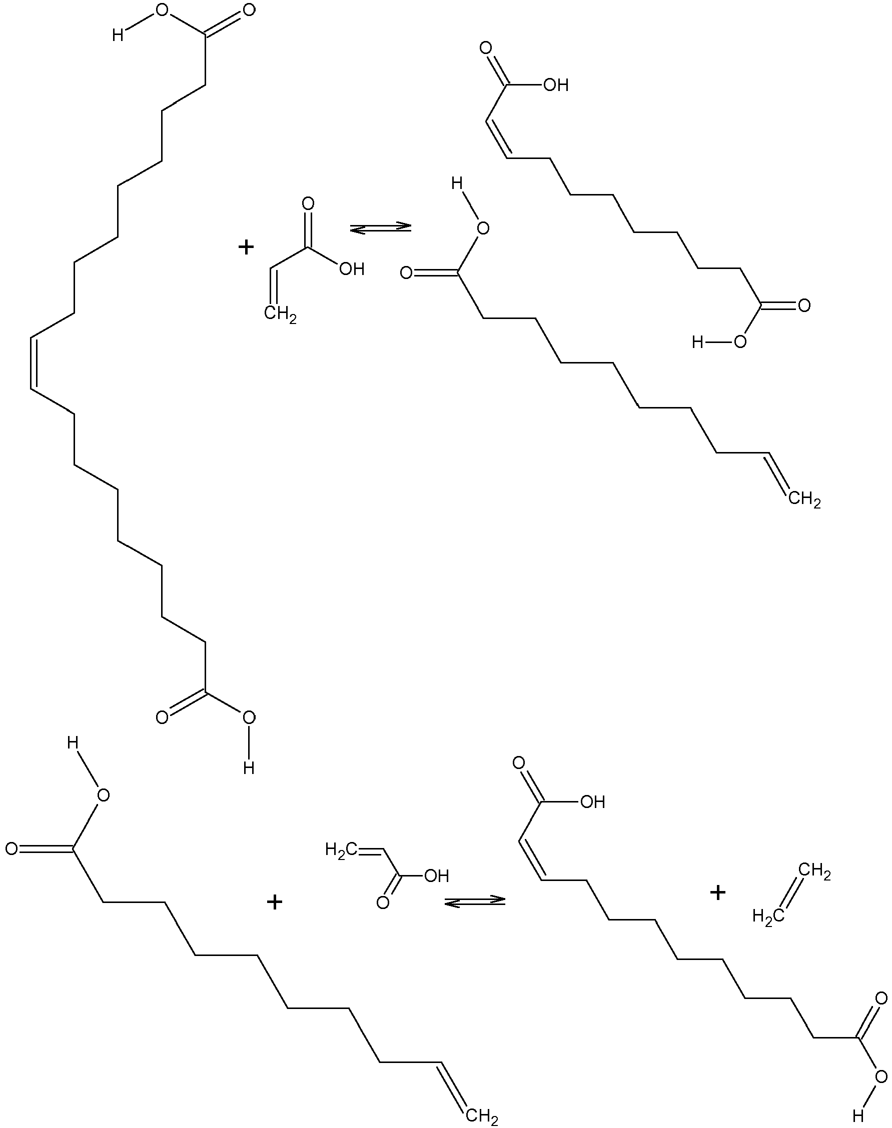

Dans certaines conditions de fermentation l'huile de macadamia ou de l'huile d'argousier est oxydée en partie en un diacide en C16, l'acide α-ω 7-hexadécènedioique à partir de l'acide palmitoléique contenu dans ces huiles.

Le processus réactionnel de cette deuxième étape mettant en oeuvre ce diacide et l'acide acrylique pour produire deux diacides différents peut être décrit par le mécanisme suivant :

HOOC-(CH₂)₅-CH=CH-(CH₂)₇-COOH + HOOC-CH=CH₂ ⇔ HOOC-CH=CH-(CH₂)₇-COOH + CH₂=CH-(CH₂)₅-COOH

et par réaction consécutive avec l'acide acrylique utilisé en excès :

CH₂=CH-(CH₂)₅-COOH + HOOC-CH=CH₂ ⇔ HOOC-CH=CH-(CH₂)₅-COOH + CH₂=CH₂.

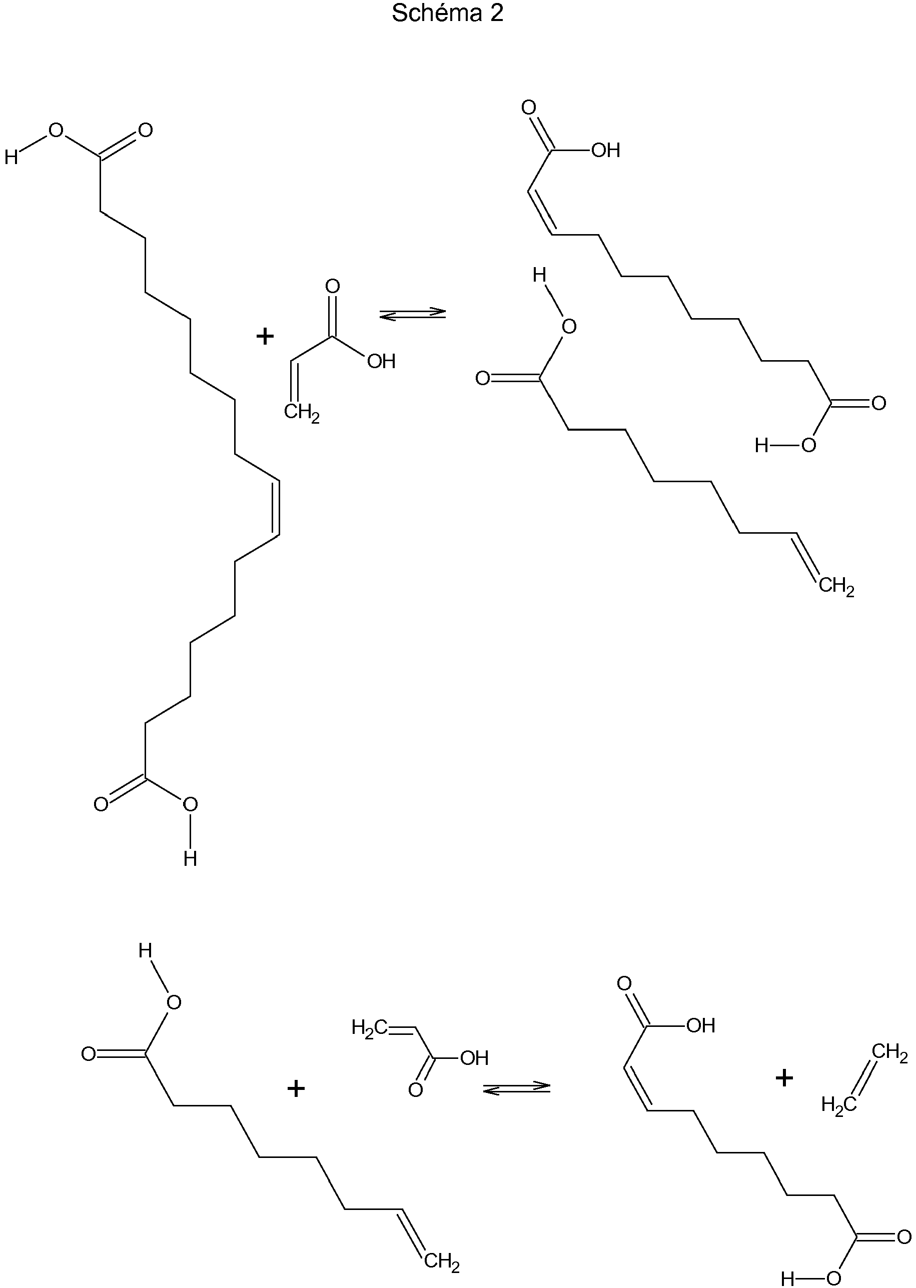

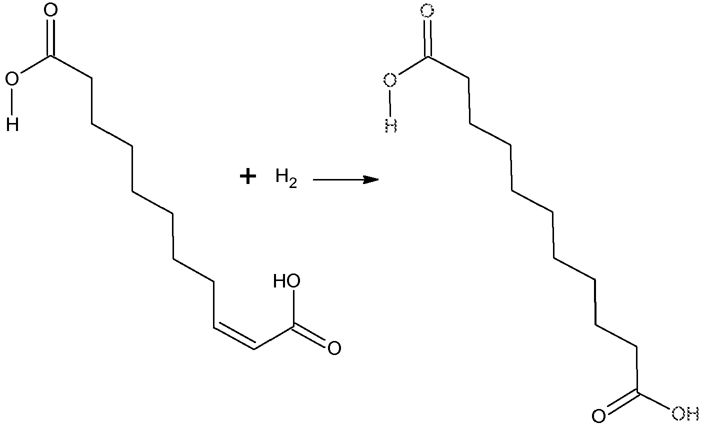

L'acide palmitoléique peut être converti lors de la fermentation en l'acide α-ω 7-hexadécènedioique. La deuxième étape du procédé va conduire à deux α-ω diacides de longueurs différentes selon le processus suivant :

HOOC-(CH₂)₅-CH=CH-(CH₂)₇-COOH + HOOC-CH=CH₂ ⇔ HOOC-CH=CH-(CH₂)₇-COOH + CH₂=CH-(CH₂)₅-COOH

et par réaction consécutive avec l'acide acrylique utilisé en excès :

CH₂=CH-(CH₂)₅-COOH + HOOC-CH=CH₂ ⇔ HOOC-CH=CH-(CH₂)₅-COOH + CH₂=CH₂

Ces deux acides peuvent être hydrogénés pour former des diacides en C11 et C9 respectivement.

L'acide pétroselinique est converti lors de la fermentation en l'acide α-ω 6-octadécènedioique. La deuxième étape du procédé va conduire à deux α-ω diacides de longueurs différentes selon le processus suivant :

HOOC-(CH₂)₄-CH=CH-(CH₂)₁₀-COOH + HOOC-CH=CH₂ ⇔ HOOC-CH=CH-(CH₂)₄-COOH + CH₂=CH-(CH₂)₁₀-COOH

et par réaction consécutive avec l'acide acrylique utilisé en excès :

CH₂=CH-(CH₂)₁₀-COOH + HOOC-CH=CH₂ ⇔ HOOC-CH=CH-(CH₂)₁₀-COOH + CH₂=CH₂

L'acide gadoléléique est converti lors de la fermentation en l'acide α-ω 9-eicosènedioique. La deuxième étape du procédé va conduire à deux α-ω diacides de longueurs différentes selon le processus suivant.

HOOC-(CH₂)₇-CH=CH-(CH₂)₉-COOH + HOOC-CH=CH₂ ⇔ HOOC-CH=CH-(CH₂)₇-COOH + CH₂=CH-(CH₂)₉-COOH

et par réaction consécutive avec l'acide acrylique utilisé en excès :

CH₂=CH-(CH₂)₉-COOH + HOOC-CH=CH₂ ⇔ HOOC-CH=CH-(CH₂)₉-COOH + CH₂=CH₂

Le procédé de l'invention est illustré par les exemples ci-après.

### EXEMPLES

### Exemple 1 : Fermentation de l'acide oléique.

Dans cet exemple.on cultivera une levure comportant des enzymes oxygénases à pH=7, dans un milieu d'eau désionisée contenant du sorbitol, des oligoéléments, de l'urée de l'acide oléique. Le mélange sera ensuite stérilisé à 120°C pendant 15 minutes. Une souche de levure sera ensuite inoculée au milieu de culture. La culture sera maintenue à 30°C. Une solution de soude sera ajoutée en continu pour maintenir le pH à 7 - 7,5. Après 48 heures de culture, le diacide insaturé sera récupéré par extraction au diéthylether. Après élimination du solvant par évaporation, on récupérera des cristaux qui après recristallisation ont un point de fusion de 69°C, c'est-à-dire équivalent à celui décrit pour l'acide 9-octadécènedioique.

### Exemple 2 : Fermentation de l'acide érucique

L'exemple 1 sera reproduit avec de l'acide érucique. On obtiendra une de l'acide 9-docosènedioique.

### Exemple 3: Métathèse croisée de l'acide 9-octadécènedioique avec l'acide acrylique.

Cet exemple illustre la synthèse du diacide en C11 à partir de l'acide 9-octadécènedioique obtenu dans l'exemple 1 dans une deuxième étape consistant en une métathèse croisée avec l'acide acrylique. On utilisera pour cette réaction un catalyseur complexe de type bispyridine ruthénium analogue à celui décrit dans la publication de Chen-Xi Bai et al., Org. Biomol. Chem., (2005), 3, 4139-4142. La réaction sera effectuée dans CH₂Cl₂, avec une concentration molaire d'acide acrylique deux fois supérieure à celle de l'acide 9-octadécénedioique une température de l'ordre de 80°C, et pendant 12 heures, en présence du catalyseur à une concentration de 1 % mole par rapport à l'acide 9-octadécènedioique. Le produit obtenu, sous sa forme ester ou acide, pourra être hydrogéné selon un processus classique avec un rendement de 100%.

### Exemple 4 : Métathèse croisée de l'acide 9-docosènedioique.

On reproduira l'exemple 3 avec le diacide de l'exemple 2, l'acide 9-docosènedioique. On obtiendra un rendement sensiblement équimolaire en acides 2-undécènedioique et 2-pentadécènedioique.

### Exemple 5 : métathèse croisée du diester C18 insaturé avec l'acrylate de méthyle

Dans un tube de Schlenk de 50 ml purgé à l'azote, on charge 170 mg de 9-octadécènedioate de méthyle (0,5 mmol), 170 mg d'acrylate de méthyle (2 mmol) et 10 ml de toluène distillé sur sodium benzophénone On chauffe à 100°C, puis sous agitation magnétique, on ajoute avec une seringue et un pousse seringue 0,3 mg (0,5.10-3 mmol) de catalyseur de Hoveyda-Grubbs de seconde génération (1,3-bis(2,4,6-triméthylphényl)-2-imidazolidinylidène) dichloro(o-isopropoxyphénylméthylène)ruthénium) disponible auprès de la société ALDRICH, dissous dans 2 ml de toluène sur une période de 2h. A la fin de l'ajout, on laisse réagir 4 heures. Le mélange réactionnel est analysé par chromatographie en phase gaz. La conversion du diester un saturé est supérieure à 99%. Le rendement en 2-undécènedioate de méthyle est de 95%.

## Revendications

1. Procédé de synthèse de diacides ou de diesters de formule générale : ROOC-(CH₂)ₙ-(CH=CH)ₐ-(CH₂)ₘCOOR₁ dans laquelle n et m différents représentent chacun un nombre entier tel que leur somme est comprise entre 6 et 15, a un indice égal à 0 ou 1 et R et R₁ sont soit H, soit un radical alkyle de 1 à 4 atomes de carbone, à partir d'acides ou d'esters gras naturels à longue chaîne mono-insaturés comportant au moins 10 atomes de carbone adjacents par molécule, de formule CH₃-(CH₂)ₚ-CH=CH-(CH₂)_{q}-COOR, dans laquelle R représente H ou un radical alkyle comportant de 1 à 4 atomes de carbone, et p et q identiques ou différents, sont des indices compris entre 2 et 11, consistant:
- dans une première étape, à oxyder par fermentation par un micro organisme comportant des enzymes *Oxygenases* tel qu'une bactérie, un champignon ou une levure ledit acide ou ester gras naturel en au moins un diacide ou diester carboxylique mono-insaturé puis à soumettre
- dans une deuxième étape le produit de la première étape à une métathèse croisée avec un composé de formule R₂OOC-(CH₂)ₓ-CH=CH-R₃ dans laquelle R₂ est soit H, soit un radical alkyle comportant de 1 à 4 atomes de carbone, x est soit 0, soit 1 soit 2 et R₃ est H, CH₃ ou COOR₂ formant dans ce dernier cas une molécule cyclique ou non, et un catalyseur à base de ruthénium, pour obtenir un composé insaturé de formule ROOC-(CH₂)_{q}-CH=CH-(CH₂)ₓ-COOR₂, puis dans une troisième étape, facultative, enfin à transformer par hydrogénation de la double liaison le composé insaturé en composé saturé.

2. Procédé selon la revendication 1 **caractérisé en ce que** la première étape de fermentation est réalisée en présence une souche de *Candida Tropicalis* contenant les enzymes Cytochrome P 450 Monooxygenase.

3. Procédé selon l'une des revendications 1 ou 2 **caractérisé en ce que** la deuxième étape de métathèse est réalisée avec l'acide acrylique.

4. Procédé selon l'une des revendications à 3 dans lequel le catalyseur de deuxième étape est à base de ruthénium et de rhénium.

5. Procédé selon l'une des revendications 1 à 4 **caractérisé en ce que** l'on synthétise l'acide 2-undécènedioique de formule HOOC -CH=CH -(CH₂)₇COOH à partir de l'acide oléique.

6. Procédé selon l'une des revendications 1 à 4 **caractérisé en ce que** l'on synthétise les acides 2-undécènedioique et 2-pentadécènedioique à partir de l'acide érucique.

## Patentansprüche

1. Verfahren zur Synthese von Disäuren oder Diestern der allgemeinen Formel: ROOC-(CH₂)ₙ-(CH=CH)ₐ-(CH₂)ₘCOOR₁, worin n und m verschieden sind, jeweils für eine solche ganze Zahl stehen, dass ihre Summe zwischen 6 und 15 liegt, a für einen Index mit einem Wert von 0 oder 1 steht und R und R₁ entweder für H oder einen Alkylrest mit 1 bis 4 Kohlenstoffatomen stehen, aus natürlichen langkettigen einfach ungesättigten Fettsäuren oder Fettestern mit mindestens 10 benachbarten Kohlenstoffatomen pro Molekül der Formel CH₃-(CH₂)ₚ-CH=CH-(CH₂)_{q}-COOR, worin R für H oder einen Alkylrest mit 1 bis 4 Kohlenstoffatomen steht und p und q gleich oder verschieden sind und für Indices zwischen 2 und 11 stehen, bei dem man:
- in einem ersten Schritt die natürliche Fettsäure bzw. den natürlichen Fettester durch Fermentation durch einen Mikroorganismus, der Oxygenase-Enzyme umfasst, wie ein Bakterium, einen Pilz oder eine Hefe, zu mindestens einer einfach ungesättigten Dicarbonsäure bzw. einem einfach ungesättigten Dicarbonsäureester oxidiert und dann
- in einem zweiten Schritt das Produkt aus dem ersten Schritt einer Kreuzmetathese mit einer Verbindung der Formel R₂OOC- (CH₂) ₓ-CH=CH-R₃, worin R₂ entweder für H oder für einen Alkylrest mit 1 bis 4 Kohlenstoffatomen steht, x entweder für 0 oder für 1 oder für 2 steht und R₃ für H, CH₃ oder COOR₂ steht, wobei sich in diesem letzteren Fall ein cyclisches oder acyclisches Molekül ergibt, und einem Rutheniumkatalysator unterwirft, wobei man eine ungesättigte Verbindung der Formel ROOC-(CH₂)_{q}-CH=CH-(CH₂)ₓ-COOR₂ erhält, und dann in einem fakultativen dritten Schritt schließlich die ungesättigte Verbindung durch Hydrierung der Doppelbindung in eine gesättigte Verbindung umwandelt.

2. Verfahren nach Anspruch 1, **dadurch gekennzeichnet, dass** der erste Schritt der Fermentation in Gegenwart eines *Candida*-*Tropicalis*-Stamms, der Cytochrom-P450-Monooxygenase-Enzyme enthält, durchgeführt wird.

3. Verfahren nach Anspruch 1 oder 2, **dadurch gekennzeichnet, dass** der zweite Schritt der Metathese mit Acrylsäure durchgeführt wird.

4. Verfahren nach einem der Ansprüche 1 bis 3, bei dem der Katalysator des zweiten Schritts auf Ruthenium und Rhenium basiert.

5. Verfahren nach einem der Ansprüche 1 bis 4, **dadurch gekennzeichnet, dass** man 2-Undecendisäure der Formel HOOC-CH=CH-(CH₂)₇COOH aus Ölsäure synthetisiert.

6. Verfahren nach einem der Ansprüche 1 bis 4, **dadurch gekennzeichnet, dass** man 2-Undecendisäure und 2-Pentadecendisäure aus Erucasäure synthetisiert.

## Claims

1. A process for the synthesis of diacids or diesters of general formula ROOC-(CH₂)ₙ-(CH=CH)ₐ-(CH₂)ₘCOOR₁, in which n and m different, each represent an integer such that their sum is between 6 and 15, a is an index equal to 0 or 1 and R and R₁ are either H or an alkyl radical comprising from 1 to 4 carbon atoms, starting from long-chain natural monounsaturated fatty acids or esters comprising at least 10 adjacent carbon atoms per molecule, of formula CH₃-(CH₂)ₚ-CH=CH-(CH₂)_{q}-COOR, in which R represents H or an alkyl radical comprising from 1 to 4 carbon atoms and p and q, which are identical or different, are indices between 2 and 11, which consists:
- in a first stage, in oxidizing, by fermentation by a microorganism comprising oxygenase enzymes, such as a bacterium, a fungus or a yeast, said natural fatty acid or ester to give at least one monounsaturated dicarboxylic acid or dicarboxylate, then,
- in a second stage, in subjecting the product from the first stage to cross-metathesis with a compound of formula R₂OOC-(CH₂)ₓ-CH=CH-R₃, in which R₂ is either H or an alkyl radical comprising from 1 to 4 carbon atoms, x is either 0 or 1 or 2 and R₃ is H, CH₃ or COOR₂, in the last case forming a cyclic or noncyclic molecule, and a catalyst based on ruthenium in order to obtain an unsaturated compound of formula ROOC-(CH₂)_{q}-CH=CH-(CH₂)ₓ-COOR₂, and then, in an optional third stage, in finally converting, by hydrogenation of the double bond, the unsaturated compound to give a saturated compound.

2. The process as claimed in claim 1, **characterized in that** the first fermentation stage is carried out in the presence of a *Candida tropicalis* strain comprising cytochrome P450 monooxygenase enzymes.

3. The process as claimed in either of claims 1 and 2, **characterized in that** the second metathesis stage is carried out with acrylic acid.

4. The process as claimed in one of claims 1 to 3, in which the catalyst of the second stage is based on ruthenium and rhenium.

5. The process as claimed in one of claims 1 to 4, **characterized in that** 2-undecenedioic acid of formula HOOC-CH=CH-(CH₂)₇COOH is synthesized starting from oleic acid.

6. The process as claimed in one of claims 1 to 4, **characterized in that** 2-undecenedioic acid and 2-pentadecenedioic acid are synthesized starting from erucic acid.
